# EUROPÄISCHE PATENTANMELDUNG

(11) **EP 1 555 012 A1**
(43) Veröffentlichungstag der Anmeldung: **20.07.2005**
(21) Anmeldenummer: 04030232.5
(22) Anmeldetag: 21.12.2004
(51) Int. Cl.: A61K 7/13

(54) **Anthrachinon enthaltende Haarfärbemittel**

(30) Priorität: 10.01.2004 DE 102004001522
(71) Anmelder: Clariant GmbH, 65929 Frankfurt am Main (DE)
(72) Erfinder: Meder, Markus, Dr., 97956 Werbach (DE); Schoefberger, Georg, Dr., 4052 Basel (CH)
(74) Vertreter: Mikulecky, Klaus, Dr.

(57) **Zusammenfassung**

Es werden Haarfärbemittel beschrieben enthaltend eine oder mehrere Anthrachinonverbindungen der Formel I 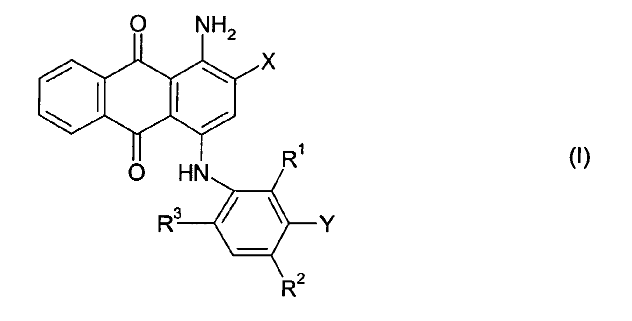 worin
- R¹, R² und R³: jeweils unabhängig voneinander für Wasserstoff, für eine lineare oder verzweigte Alkylgruppe mit 1 bis 22 Kohlenstoffatomen, für eine lineare oder verzweigte Alkenylgruppe mit 2 bis 22 Kohlenstoffatomen oder für eine Gruppe N(R⁴)C(O)R⁵ stehen,
- R⁴ und R⁵: unabhängig voneinander Wasserstoff oder eine Alkylgruppe mit 1 bis 22 Kohlenstoffatomen bedeuten,
- X: SO₃A¹ oder OCH₂CH₂(OCH₂CH₂)ₓOCH₃ bedeutet,
- Y: H oder SO₃A² ist,
- A¹ und A²: jeweils unabhängig voneinander H⁺, Li⁺, Na⁺, K⁺, Mg²⁺/2, Ca²⁺/2, Al³⁺/3 oder ⁺NR⁶R⁷R⁸R⁹ bedeuten,
- R⁶, R⁷, R⁸, R⁹: jeweils unabhängig voneinander Wasserstoff, einen Alkylrest mit 1 bis 22 Kohlenstoffatomen oder einen Hydroxyalkylrest mit 2 bis 10 Kohlenstoffatomen bedeuten, und
- x: für eine Zahl von 0 bis 10 steht,
dadurch gekennzeichnet, dass die Verbindungen der Formel I genau eine Gruppe ausgewählt aus SO₃A¹ oder SO₃A² enthalten.

## Beschreibung

Die Erfindung betrifft Mittel zum Färben und Tönen von menschlichen Haaren enthaltend Anthrachinonverbindungen.

Anthrachinone stellen eine wichtige Verbindungsklasse an Direktfarbstoffen dar und werden insbesondere zum Färben von Textilien eingesetzt. Auch zum Haarfärben haben sich neben den Oxidationsfarbstoffen Direktfarbstoffe zum Erzielen einer breiten Farbpalette von Gelb-, Orange, Rot-, Blau-, Grün- und Violettfärbungen bewährt.

In DE 33 13 337 werden Anthrachinonverbindungen mit zwei Sulfonsäuregruppen und deren Verwendung zum Färben von Fasern, insbesondere von Polyamiden, beschrieben. Das Aufziehen dieser Verbindungen auf menschliche Haare ist allerdings unzureichend und das Färbeergebnis unbefriedigend.

In US 5,595,197 wird ein Verfahren zum Färben menschlicher Haare beschrieben, wobei ein sulfonsäuregruppenhaltiger Farbstoff in einem Wasserdampfgemisch bei mindestens 75°C aufgebracht werden muss um gute Färberesultate zu erhalten.

In DE 202 13 695 U1 wird ein Haarfärbemittel enthaltend einen blauen Anthrachinonfarbstoff in Kombination mit mindestens einem weiteren direktziehenden Farbstoff offenbart.

Es bestand jedoch weiterhin ein großer Bedarf nach direktziehenden Farbstoffen zum Färben von Keratinfasern, insbesondere von menschlichen Haaren, die toxikologisch unbedenklich sind und Färbungen bei milden Bedingungen in der gewünschten Intensität ermöglichen. Weiterhin wird für die erzielten Haarfärbungen eine gute Licht-, Dauerwell-, Säure- und Reibeechtheit gefordert. Die Haarfärbungen müssen ohne Einwirkung von Licht, Reibung und chemischen Mitteln über einen Zeitraum von mindestens 4 bis 6 Wochen stabil bleiben. Darüber hinaus sollten direktziehende Farbstoffe auch in oxidativen Haarfärbemitteln anwendbar sein.

Überraschend wurde gefunden, dass Anthrachinonverbindungen Formel I 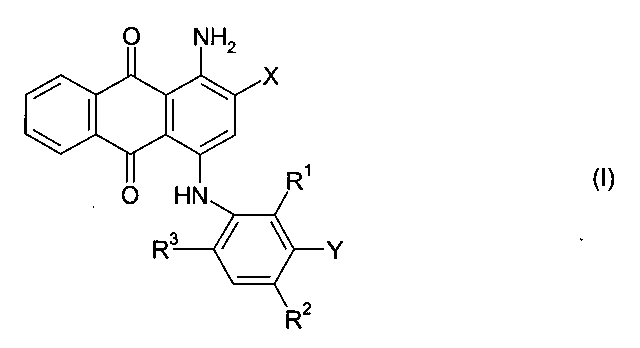 worin
- R¹, R² und R³: jeweils unabhängig voneinander für Wasserstoff, für eine lineare oder verzweigte Alkylgruppe mit 1 bis 22, vorzugsweise 1 bis 8, besonders bevorzugt 1 bis 4, Kohlenstoffatomen, für eine lineare oder verzweigte Alkenylgruppe mit 2 bis 22, vorzugsweise 2 bis 8, besonders bevorzugt 2 bis 4, Kohlenstoffatomen oder für eine Gruppe N(R⁴)C(O)R⁵ stehen,
- R⁴ und R⁵: unabhängig voneinander Wasserstoff oder eine Alkylgruppe mit 1 bis 22, vorzugsweise 1 bis 8, besonders bevorzugt 1 bis 4, Kohlenstoffatomen bedeuten,
- X: SO₃A¹ oder OCH₂CH₂(OCH₂CH₂)ₓOCH₃ bedeutet,
- Y: H oder SO₃A² ist,
- A¹ und A²: jeweils unabhängig voneinander H⁺, Li⁺, Na⁺, K⁺, Mg²⁺/2, Ca²⁺/2, Al³⁺/3 oder ⁺NR⁶R⁷R⁸R⁹ bedeuten,
- R⁶, R⁷, R⁸ und R⁹: jeweils unabhängig voneinander Wasserstoff, einen Alkylrest mit 1 bis 22, vorzugsweise 1 bis 18, Kohlenstoffatomen oder einen Hydroxyalkylrest mit 2 bis 10 Kohlenstoffatomen bedeuten, und
- x: für eine Zahl von 0 bis 10, vorzugsweise 1 bis 5, besonders bevorzugt 1, steht,
dadurch gekennzeichnet, dass die Verbindungen der Formel I genau eine Gruppe ausgewählt aus SO₃A¹ oder SO₃A² enthalten,
hervorragend als Direktfarbstoffe für semipermanente und permanente Haarfärbemittel geeignet sind und sowohl als Farbgeber, aber auch in Kombination mit Oxidationsfarbstoffvorstufen als Farbnuancengeber einsetzbar sind. Zudem wurde gefunden, dass sich die Verbindungen der Formel I durch hohe Stabilität, gute Wasserlöslichkeit, brillante Farbintensität, hohe Waschbeständigkeit und hohe Lichtechtheit auszeichnen. Die Farbstoffe der Formel I haben physiologisch günstige Eigenschaften und können z.B. bei Variation der Substituenten R¹ bis R³ Farbtöne von blau bis violett liefern.

Gegenstand der vorliegenden Erfindung sind daher Haarfärbemittel enthaltend eine oder mehrere Anthrachinonverbindungen der Formel I 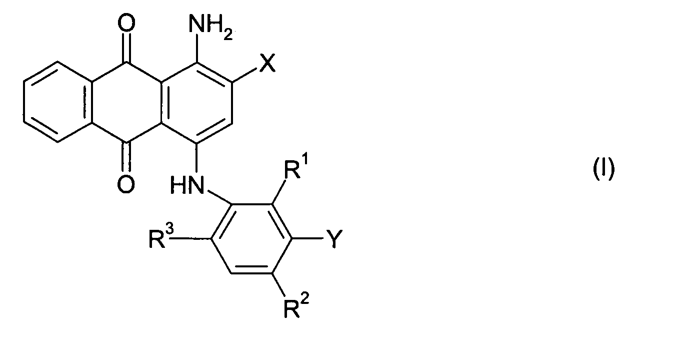 worin
- R¹, R² und R³: jeweils unabhängig voneinander für Wasserstoff, für eine lineare oder verzweigte Alkylgruppe mit 1 bis 22, vorzugsweise 1 bis 8, besonders bevorzugt 1 bis 4, Kohlenstoffatomen, für eine lineare oder verzweigte Alkenylgruppe mit 2 bis 22, vorzugsweise 2 bis 8, besonders bevorzugt 2 bis 4, Kohlenstoffatomen oder für eine Gruppe N(R⁴)C(O)R⁵ stehen,
- R⁴ und R⁵: unabhängig voneinander Wasserstoff oder eine Alkylgruppe mit 1 bis 22, vorzugsweise 1 bis 8, besonders bevorzugt 1 bis 4, Kohlenstoffatomen bedeuten,
- X: SO₃A¹ oder OCH₂CH₂(OCH₂CH₂)ₓOCH₃ bedeutet,
- Y: H oder SO₃A² ist,
- A¹ und A²: jeweils unabhängig voneinander H⁺, Li⁺, Na⁺, K⁺, Mg²⁺/2, Ca²⁺/2, Al³⁺/3 oder ⁺NR⁶R⁷R⁸R⁹, vorzugsweise H⁺, Na⁺ oder ⁺NR⁶R⁷R⁸R⁹, bedeuten,
- R⁶, R⁷, R⁸, R⁹: jeweils unabhängig voneinander Wasserstoff, einen Alkylrest mit 1 bis 22, vorzugsweise 1 bis 18, Kohlenstoffatomen oder einen Hydroxyalkylrest mit 2 bis 10 Kohlenstoffatomen bedeuten, und
- x: für eine Zahl von 0 bis 10, vorzugsweise 1 bis 5, besonders bevorzugt 1, steht,
dadurch gekennzeichnet, dass die Verbindungen der Formel I genau eine Gruppe ausgewählt aus SO₃A¹ oder SO₃A² enthalten.

Diese Haarfärbemittel sind insbesondere zum Färben von menschlichen Haaren geeignet.

Weiterer Gegenstand der Erfindung ist auch die Verwendung der erfindungsgemäßen Mittel zum Färben von Keratinfasern, insbesondere zum Färben von menschlichen Haaren.

In einer bevorzugten Ausführungsform der Erfindung enthalten die Haarfärbemittel Anthrachinonverbindungen der Formel I worin
- R¹, R² und R³: gleich sind und für Wasserstoff oder für Methyl stehen,
- X: SO₃A¹ ist,
- A¹: H⁺, Li⁺, Na⁺, K⁺, Mg²⁺/2, Ca²⁺/2, Al³⁺/3 oder ⁺NR⁶R⁷R⁸R⁹, vorzugsweise H⁺, Na⁺ oder ⁺NR⁶R⁷R⁸R⁹, bedeutet,
- R⁶, R⁷, R⁸ und R⁹: jeweils unabhängig voneinander Wasserstoff, einen Alkylrest mit 1 bis 22, vorzugsweise 1 bis 18, Kohlenstoffatomen oder einen Hydroxyalkylrest mit 2 bis 10 Kohlenstoffatomen bedeuten, und
- Y: H bedeutet.

In einer weiteren bevorzugten Ausführungsform der Erfindung enthalten die Haarfärbemittel Anthrachinonverbindungen der Formel I worin
- R¹ und R³: Wasserstoff sind,
- R²: tert.-Butyl, n-Butyl oder -N(H)C(O)CH₃ bedeutet,
- X: SO₃A¹ ist,
- A¹: H⁺, Li⁺, Na⁺, K⁺, Mg²⁺/2, Ca²⁺/2, Al³⁺/3 oder ⁺NR⁶R⁷R⁸R⁹, vorzugsweise H⁺, Na⁺ oder ⁺NR⁶R⁷R⁸R⁹, bedeutet,
- R⁶, R⁷, R⁸ und R⁹: jeweils unabhängig voneinander Wasserstoff, einen Alkylrest mit 1 bis 22, vorzugsweise 1 bis 18, Kohlenstoffatomen oder einen Hydroxyalkylrest mit 2 bis 10 Kohlenstoffatomen bedeuten, und
- Y: H bedeutet.

In einer weiteren bevorzugten Ausführungsform der Erfindung enthalten die Haarfärbemittel Anthrachinonverbindungen der Formel I worin
- R¹, R² und R³: für Methyl stehen,
- X: OCH₂CH₂(OCH₂CH₂)ₓOCH₃ bedeutet,
- x: 1 ist,
- Y: SO₃A² bedeutet,
- A²: H⁺, Li⁺, Na⁺, K⁺, Mg²⁺/2, Ca²⁺/2, Al³⁺/3 oder ⁺NR⁶R⁷R⁸R⁹, vorzugsweise H⁺, Na⁺ oder ⁺NR⁶R⁷R⁸R⁹, bedeutet, und
- R⁶, R⁷, R⁸ und R⁹: jeweils unabhängig voneinander Wasserstoff, einen Alkylrest mit 1 bis 22, vorzugsweise 1 bis 18, Kohlenstoffatomen oder einen Hydroxyalkylrest mit 2 bis 10 Kohlenstoffatomen bedeuten.

Die erfindungsgemäßen Mittel können zum semipermanenten oder permanenten Färben von Keratinfasern, vorzugsweise menschlichen Haaren, verwendet werden.

Die erfindungsgemäßen Mittel können die Anthrachinonverbindungen der Formel I als alleinige Farbkomponente als Einzelsubstanz oder als Gemisch enthalten.

In einer weiteren bevorzugten Ausführungsform der Erfindung enthalten die erfindungsgemäßen Mittel zur Erzielung weiterer Farbnuancen neben den Anthrachinonverbindungen der Formel I aber auch ein oder mehrere weitere Direktfarbstoffe.

Bevorzugte Direktfarbstoffe sind Nitroanilinderivate, wie 1-[(2-Hydroxyethyl)-amino]-2-nitrobenzol (Velsol™ Yellow 2), 4-Hydroxypropylamino-3-Nitrophenol (Velsol™ Red BN), 3-Nitro-p-Hydroxyethylaminophenol (Velsol™ Red 54), 4-Hydroxyethylamino-3-Nitroanilin (Velsol™ Red 3), N,N'-Bis-(Hydroxyethyl)-2-Nitro-p-Phenylendiamine (Velsol™ Violet BS), N,N',N'-Tris-(Hydroxyethyl)-2-Nitro-p-Phenylendiamine (Velsol™ Blue 2), 4-(2'-Hydroxyethyl)-amino-3-nitro-toluol, 4-(2'-Hydroxyethyl)-amino-3-nitrobenzylalkohol, 4-(2'-Hydroxyethy!)-amino-3-nitro-1 -triftuormethy)-benzo), 4-(2',3'-Dihydroxypropyl)-amino-3-nitro-chlorbenzol, 4-(2'-Hydroxyethyl)-amino-3-nitrobrombenzol und 4-(2',3'-Dihydroxypropyl)-amino-3-nitro-brombenzol, Nitrobenzolderivate, beispielsweise 2-Amino-4-nitro-phenol, Pikraminsäure, 1-[(2'-Hydroxyethyl)-amino]-2-amino-4-nitrobenzol, 2-Nitro-4-[(2'-hydroxyethyl)amino]-anilin, 4-Bis-[(2'-hydroxyethyl)amino]-1-methylamino-2-nitro-benzol, 2,5-Bis-[(2'-hydroxyethyl)-amino]-nitrobenzol, 2-(2'-hydroxyethyl)-amino-4,6-dinitro-phenol, 1-Amino-4-(2',3'dihydroxypropyl)-amino-2-nitro-5-chlor-benzol, aber auch Triphenylmethanfarbstoffe, wie zum Beispiel Basic Violet 1 (C.I. 42535), Azofarbstoffe, wie zum Beispiel Acid Brown 4 (C.I. 14805), Anthrachinonfarbstoffe, die nicht durch Formel I definiert sind, wie zum Beispiel Disperse Blue 23 (C.I. 61545), Disperse Violet 4 (C.I. 61105), 1,4,5,8-Tetraaminoanthrachinon und 1,4-Diaminoanthrachinon, sowie die in DE 202 13 695 U1 genannten direktziehenden Farbstoffe.

Der pH-Wert der nicht-oxidativen Haarfärbemittel liegt vorzugsweise im Bereich von 2 bis 7, besonders bevorzugt im Bereich von 4 bis 6.

Die Herstellung der Anthrachinonverbindungen der Formel I kann nach Methoden erfolgen, die dem Fachmann bekannt sind, z.B. nach den in den Schriften CH 194 097, US 2,117,569 und DE 642 726 beschriebenen Methoden.

Die Haarfärbemittel können zusätzlich zu der einen oder den mehreren Anthrachinonverbindungen der Formel I Oxidationsfarbstoffvorstufen enthalten und zur permanenten Färbung von Keratinfasern, insbesondere menschlichem Haar, eingesetzt werden.

In einer weiteren bevorzugten Ausführungsform der Erfindung enthalten die erfindungsgemäßen Mittel neben den Anthrachinonverbindungen der Formel I und gegebenenfalls den weiteren Direktfarbstoffen somit auch ein oder mehrere Oxidationsfarbstoffvorstufen.

Bei den Oxidationsfarbstoffvorstufen handelt es sich vorzugsweise um p-Phenylendiamine und p-Aminophenole oder deren Derivate wie beispielsweise p-Toluylendiamin, p-Phenylendiamin, p-Aminophenol, die zum Zweck der Nuancierung der Färbung mit sogenannten Modifiern oder Kupplern, wie zum Beispiel m-Phenylendiamin, Resorcin, m-Aminophenol und deren Derivaten kombiniert werden.

Die erfindungsgemäßen Haarfärbemittel enthalten die Anthrachinonverbindungen der Formel I, bezogen auf die fertigen Haarfärbemittel, vorzugsweise in Mengen von 0,001 bis 15 Gew.-%, besonders bevorzugt in Mengen von 0,05 bis 5 Gew.-% und insbesondere bevorzugt in Mengen von 0,1 bis 3 Gew.-%.

Sofern in den erfindungsgemäßen Haarfärbemitteln neben den Anthrachinonverbindungen der Formel I ein oder mehrere weitere Direktfarbstoffe enthalten sind, enthalten die erfindungsgemäßen Haarfärbemittel die Anthrachinonverbindungen der Formel I und die weiteren Direktfarbstoffe zusammen, bezogen auf die fertigen Haarfärbemittel, vorzugsweise in Mengen von 0,001 bis 15 Gew.-%, besonders bevorzugt in Mengen von 0,05 bis 5 Gew.-% und insbesondere bevorzugt in Mengen von 0,1 bis 3 Gew.-%.

Sofern in den erfindungsgemäßen Haarfärbemitteln ein oder mehrere Oxidationsfarbstoffvorstufen enthalten sind, enthalten die erfindungsgemäßen Haarfärbemittel die Anthrachinonverbindungen der Formel I und die Oxidationsfarbstoffvorstufen zusammen, bezogen auf die fertigen Haarfärbemittel, vorzugsweise in Mengen von 0,01 bis 15 Gew.-%, besonders bevorzugt in Mengen von 0,1 bis 5 Gew.-% und insbesondere bevorzugt in Mengen von 0,5 bis 3 Gew.-%.

Sofern in den erfindungsgemäßen Haarfärbemitteln neben den Anthrachinonverbindungen der Formel I ein oder mehrere weitere Direktfarbstoffe und ein oder mehrere Oxidationsfarbstoffvorstufen enthalten sind, enthalten die erfindungsgemäßen Haarfärbemittel die Anthrachinonverbindungen der Formel I, die weiteren Direktfarbstoffe und die Oxidationsfarbstoffvorstufen zusammen, bezogen auf die fertigen Haarfärbemittel, vorzugsweise in Mengen von 0,01 bis 15 Gew.-%, besonders bevorzugt in Mengen von 0,1 bis 5 Gew.-% und insbesondere bevorzugt in Mengen 0,5 bis 3 Gew.-%.

Als Oxidationsmittel zur Entwicklung der Haarfärbungen kommen vorzugsweise Hydrogenperoxyd und dessen Additionsverbindungen in Betracht.

Der pH-Wert dieser oxidativen Färbemittel liegt vorzugsweise im Bereich von 2 bis 11, besonders bevorzugt im Bereich von 7 bis 10. Die Einstellung eines alkalischen pH-Wertes erfolgt vorzugsweise mit Ammoniak oder mit organischen Aminen, beispielsweise Monoethanolamin oder Triethanolamin. Für saure Einstellungen kommen vorzugsweise Zitronensäure, Essigsäure, Weinsäure in Betracht.

Zur Erhöhung der Farbintensität können die erfindungsgemäßen Mittel die in kosmetischen Systemen üblichen Carrier enthalten, vorzugsweise Benzylalkohol, Vanillin (4-Hydroxy-3-methoxy-benzaldehyd), Isovanillin, p-Hydroxyanisol, 3-Hydroxy-4-methoxy-benzaldehyd, 2-Phenoxyethanol, Salicylaldehyd, 3,5-Dihydroxybenzaldehyd, 3,4-Dihydroxybenzaldehyd, 4-Hydroxyphenylacetamid, p-Hydroxybenzoesäuremethylester, p-Hydroxybenzaldehyd, m-Kresol, Hydrochinonmonomethylether, o-Fluorphenol, m-Fluorphenol, p-Fluorphenol, 2-(2'-Hydroxyphenoxy)-ethanol, 3,4-Methylendioxyphenol, Resorcinmonomethylether, 3,4-Dimethoxyphenol, 3-Trifluormethylphenol, Resorcinmonoacetat, Ethylvanillin, 2-Thiophenethanol, Milchsäurebutylester und Glycolsäurebutylester.

Die erfindungsgemäßen Haarfärbemittel können vorteilhafterweise ein oder mehrere perlglanzgebende Verbindungen enthalten, vorzugsweise Fettsäuremonoalkanolamide, Fettsäuredialkanolamide, Monoester oder Diester von Alkylenglykol, insbesondere Ester von Ethylenglykol und/oder Propylenglykol oder deren Oligomeren mit höheren Fettsäuren, z.B. Palmitinsäure, Stearinsäure oder Behensäure oder Mischungen davon, Mono- oder Diester von Alkylenglykolen mit Fettsäuren, Fettsäuren und deren Metallsalze, Monoester oder Polyester von Glycerin mit Carbonsäuren und Ketosulfone verschiedener Art, besonders bevorzugt Ethylenglykoldistearat und Polyethylenglykoldistearat mit ca. 3 Glykoleinheiten.

Glitter- und Glanzeffekte der erfindungsgemäßen Mittel lassen sich vorzugsweise durch Zugabe von Glimmer, colorierten Polyacrylestern und Glimmer, Glimmer-Eisenoxid, Glimmer-Titanoxid und durch Zugabe von Pigmenten erzeugen. Als Pigmente eignen sich Metalloxide, beispielsweise Eisenoxide, Titanoxid, Ultramarinblau, sowie mit kationischen Coatinghüllen modifizierte Pigmente, wie in WO 00/12053 und EP 504 066 beschrieben.

Die erfindungsgemäßen Haarfärbemittel können Zusatzstoffe enthalten, die für die gefärbten Haare konditionierend und farberhaltend wirken, beispielsweise kationische und nichtionische Polymere.

Geeignete kationische Polymere sind neben den altbekannten quaternären Cellulosederivaten des Typs "Polymer JR" die unter der INCI-Bezeichnung "Polyquaternium" bekannten Verbindungen, insbesondere Polyquaternium-31, Polyquaternium-16, Polyquaternium-24, Polyquaternium-7, Polyquaternium-22, Polyquaternium-39, Polyquaternium-28, Polyquaternium-2, Polyquaternium-10, Polyquaternium-11, Polyquaternium 37&mineral oil&PPG trideceth (Salcare® SC95), Guarhydroxypropyltriammoniumchloride, sowie Calciumalginat und Ammoniumalginat.

Des weiteren können eingesetzt werden Aminosilicone, Copolymere von Diallylammoniumsalzen und Acrylamiden; quaternierte Vinylpyrrolidon/Vinylimidazol-Polymere, beispielsweise Copolymerisate aus Vinylpyrrolidon und Vinylimidazoliniummethochlorid, die unter dem Handelsnamen "Luviquat" angeboten werden; Kondensationsprodukte von Polyglykolen und Aminen; quaternierte Kollagenpolypeptide; quaternierte Weizenpolypeptide; Polyethylenimine; kationische Siliconpolymere, wie z.B. Amidomethicone; Copolymere der Adipinsäure und Dimethylaminohydroxy-propyldiethylentriamin; Polyaminopolyamid und kationische Chitinderivate, wie beispielsweise Chitosan, aber auch quaternisierte Homo- und Copolymere des Dimethyldiallylammoniumchlorids, wie sie unter dem Handelsnamen "Merquat" im Handel sind, quaternäre Vinylpyrrolidon-Copolymere, insbesondere mit Dialkylaminoalkyl(meth)acrylaten, wie sie unter dem Namen "Gafquat" bekannt sind, Polyamino-Polyamid-Derivate, beispielsweise Copolymere von Adipinsäure-Dimethylaminohydroxypropyldiethylentriamin, wie sie unter dem Namen "Cartaretine F" vertrieben werden, sowie auch bisquaternäre langkettige Ammoniumverbindungen der in der US 4,157,388 beschriebenen Harnstoff-Struktur, die unter dem Handelsnamen "Mirapol A 15" bekannt sind.

Anstelle von kationischen Polymeren oder in Kombination mit denselben können auch nichtionische Polymere verwendet werden. Als geeignete nichtionische Polymere werden vor allem Vinylpyrrolidon-Homo- und Copolymerisate, insbesondere Polyvinylpyrrolidon selbst, Copolymere aus Vinylpyrrolidon und Vinylacetat oder Terpolymerisate aus Vinylpyrrolidon, Vinylacetat und Vinylpropionat, wie sie beispielsweise von der Firma BASF unter dem Handelsnamen "Luviskol" vertrieben werden, eingesetzt.

Es können auch (Co-)Polymerisate aus den verschiedenen Acryl- und Methacrylestern, Acrylamid und Methacrylamid, beispielsweise Polyacrylamid mit Molgewichten von über 100.000 oder Dimethylhydantoin-Formaldehyd-Harze, verwendet werden.

Ebenso geeignet sind amphotäre Polymere, z.B. die unter Bezeichnung "Amphomer" vertriebenen Copolymerisate aus N-Octylacrylamid, N-Butylaminoethylmethacrylat und Acrylsäure.

Auch natürliche Polymere, wie Chitosan oder Chitosanderivate können eingesetzt werden.

Die oben genannten Polymere werden vorzugsweise in einer Menge von 0,1 bis 5 Gew.-%, besonders bevorzugt von 0,25 bis 2,5 Gew.-% insbesondere bevorzugt von 0,5 bis 1,5 Gew.-%, bezogen auf die Gesamtzusammensetzung der Haarfärbemittel, eingesetzt.

Die erfindungsgemäßen Haarfärbemittel können weitere Zusätze, beispielsweise Fette und Öle, Fettalkohole, Emulgatoren, Netzmittel, Verdicker, Weichmacher, pH-Regulatoren, Lösungsmittel, Lösungsvermittler, Konservierungsmittel oder Parfums enthalten.

Als Fette und Öle, zu denen auch Wachse zählen, können z.B. natürliche Öle wie Avocadoöl, Cocosöl, Palmöl, Sesamöl, Edrnußöl, Spermöl, Sonnenblumenöl, Mandelöl, Pfirsichkernöl, Weizenkeimöl, Macadamianußöl, Nachtkerzenöl, Jojobaöl, Ricinusöl oder auch Oliven- bzw. Sojaöl, Lanolin und dessen Derivate, sowie Mineralöle wie Paraffinöl und Vaseline verwendet werden.

Synthetische Öle und Wachse, die in den erfindungsgemäßen Mitteln verwendet werden können, sind beispielsweise Silikonöle oder Polyethylenglykole.

Weitere geeignete hydrophobe Komponenten der erfindungsgemäßen Haarfärbemittel sind insbesondere Fettalkohole, vorzugsweise solche mit 8 bis 22 Kohlenstoffatomen, beispielsweise Myristyl-, Cetyl-, Stearylalkohol, Wachsalkohole und Fettsäureester wie Isopropylmyristat, -palmitat, -stearat und -isostearat, Oleyloleat, Isocetylstearat, Hexyllaurat, Dibutyladipat, Dioctyladipat, Myristylmyristat, Oleylerucat, Polyethylenglykol- und Polyethylenglykolfettsäureester wie PEG-7-glycerylcocoat oder Cetylpalmitat.

Diese hydrophoben Komponenten sind in den erfindungsgemäßen Mitteln vorzugsweise in einer Gesamtmenge von 0,5 bis 10 Gew.-%, besonders bevorzugt von 1 bis 7,5 Gew.-% und insbesondere bevorzugt von 1,5 bis 5 Gew.-%, berechnet auf die Gesamtzusammensetzung, enthalten.

Als Lösungsmittel dienen vorzugsweise Wasser, niedere aliphatische Alkohole, beispielsweise Ethanol, Propanol, Isopropanol, Glycerin oder Glykole wie Ethylenglykol, Propylenglykol oder auch Glykolether.

Als Netzmittel und Emulgatoren stehen anionische, kationische, amphotere oder nichtionogene oberflächenaktive Substanzen wie Fettalkoholsulfate, Fettalkoholethersulfate, Alkylsulfonate, Alkylbenzolsulfate, Alkyltrimethylammoniumsalze, Alkylbetaine, oxethylierte Fettalkohole, oxethylierte Nonylphenole, Fettsäurealkanolamide und oxethylierte Fettsäureester zur Verfügung.

Höhere Fettalkohole, Bentonit, Stärke, Polyacrylsäure, Cellulosederivate wie Carboxymethylcellulose, Alginate, Vaseline, Paraffinöl und Fettsäuren können zum Verdicken der Mittel eingesetzt werden.

Netzmittel und Emulgatoren werden in Konzentrationen von 0,5 bis 30 Gew.-%, bezogen auf die fertigen Färbemittel eingesetzt, während Verdicker in einer Menge von 0,1 Gew.-% bis 25 Gew.-% enthalten sein können.

Pflegestoffe sind beispielsweise Lanolinderivate, Cholesterin, Pantothensäure, welche in Mengen von 0,1 bis 5 Gew.-% in den erfindungsgemäßen Haarbehandlungsmitteln enthalten sein können.

Bei Anwendungen der genannten Zubereitungen, bei denen die Zugabe eines Oxidationsmittels erforderlich ist, erfolgt diese in bekannter Weise, indem man die Haarfärbemittel vor der Behandlung mit dem Oxidationsmittel vermischt.

Das erfindungsgemäße Färbemittel enthaltend eine oder mehrere Anthrachinonverbindungen der Formel I oder ein Gemisch der Anthrachinonverbindungen der Formel I mit weiteren Direktfarbstoffen, gegebenenfalls kombiniert mit Oxidationsfarbstoffvorstufen wird in einer zur Färbung des Haares ausreichenden Menge der Mischung, im allgemeinen etwa 50 bis 150 ml, auf das Haar aufgetragen und verbleibt dort für etwa 1 bis 45 Minuten bei 20 bis 50°C, vorzugsweise 15 bis 30 Minuten bei ca. 40°C. Anschließend wird mit Wasser, gegebenenfalls noch mit der wässrigen Lösung einer schwachen organischen Säure, gespült und getrocknet. Als schwach organische Säuren können beispielsweise Essigsäure, Zitronensäure, Weinsäure und ähnliche Verwendung finden.

Die Zubereitungsform der erfindungsgemäßen Haarfärbemittel ist vorzugsweise eine Lösung, insbesondere eine wässrige oder eine wässrig-alkoholische Lösung, eine Emulsion, eine Dispersion, eine Creme, ein Gel oder ein Aerosolschaum oder -spray.

Die nachfolgenden Beispiele sollen den Gegenstand der Erfindung näher erläutern ohne ihn darauf einzuschränken.

### Beispiele

### Beispiel 1

Es wurden Farbmittel 1 bis 9 der im Folgenden angegebenen Zusammensetzung hergestellt.

| | |
|---|---|
| Natriumlaurylethersulfat | 7 g |
| Farbstoff gemäß Tabelle 1 | 0,5 g |
| Ethanol | 10 g |
| NaCl | q.s. |
| Wasser | ad 100 g |

### Herstellung

Die Komponenten wurden bei Raumtemperatur gemischt. Die pH-Einstellung auf ca. 5 erfolgte mittels Zitronensäure oder NaOH. Die in den Farbmitteln 1-9 verwendeten Farbstoffe und Farbstoffgemische sind in Tabelle 1 aufgeführt. Alle in Tabelle 1 genannten Farbstoffe sind käuflich erwerbbar.

**Tabelle 1**

| in den Farbmitteln 1-9 verwendete Farbstoffe und Farbstoffgemische | | | | | | | | | |
|---|---|---|---|---|---|---|---|---|---|
| Farbstoff | Farbmittel | | | | | | | | |
| (C.I. Name) | 1 | 2 | 3 | 4 | 5 | 6 | 7 | 8 | 9 |
| Velsol™ Blue 25 (Acid Blue 25) | 0,5 g | - | - | - | 0,3 g | 0,3 g | - | - | - |
| Velsol™ Blue 129 (Acid Blue 129) | - | 0,5 g | - | - | - | - | 0,3 g | - | - |
| Velsol™ Blue 230 (Acid Blue 230) | - | - | 0,5 g | - | - | - | 0,1 g | - | - |
| Velsol™ Blue 40 (Acid Blue 40) | - | - | - | 0,5 g | - | - | - | 0,2 g | - |
| VelsolT™ Violet 126 (Acid Violet 126) | - | - | - | - | 0,2 g | - | - | 0,2 g | 0,5 g |
| Velsol™ Violet BS (-) | - | - | - | - | - | 0,1 g | - | - | - |
| Velsol™ Red BN (-) | - | - | - | - | - | 0,1 g | 0,1 g | 0,1 g | - |

### Chemische Bezeichnung der Farbstoffe

| | |
|---|---|
| Velsol™ Blue 25 | Formel 1, R¹=R²=R³ = H, X=SO₃H, Y=H |
| Velsol™ Blue 129 | Formel I, R¹=R²=R³ =CH3, X=SO₃H, Y=H |
| Velsol™ Blue 230 | Formel 1, R¹=R³=H, R₂=tert.-Butyl, X=SO₃H, Y=H |
| Velsol™ Blue 40 | Formel 1, R¹=R³=H, R²=N(H)C(O)CH₃, X=SO₃H, Y=H |
| Velsol™ Violet 126 | Formel I, R¹=R²=R³ = CH₃, |
| | X=OCH₂CH₂(OCH₂CH₂)ₓOCH₃, x = 1, Y = SO₃H |
| Velsol™ Violet BS | N,N'-Bis-(2-Hydroxyethyl)-2-Nitro-p-Phenylendiamin |
| Velsol™ Red BN | 4-Hydroxypropylamino-3-nitrophenol |

### Beispiel 2

Mit den Farbmitteln 1-9 aus Beispiel 1 wurden blond gebleichte Strähnen von kaukasischem Haar gefärbt. Die Ergebnisse sind in Tabelle 2 aufgeführt.

**Tabelle 2**

| Färbung von blond gebleichten Strähnen von kaukasischem Haar mit Farbmitteln 1 bis 9 | |
|---|---|
| Farbmittel | Farbe der gefärbten Strähnen |
| 1 | blau |
| 2 | graugrün |
| 3 | grünlich |
| 4 | grünlich |
| 5 | blaugrau |
| 6 | aubergine |
| 7 | bordeaux |
| 8 | kupfer |
| 9 | aschblond |

### Beispiel 3

Die Farbmittel 1 bis 5 aus Beispiel 1 wurden auf ihre Waschbeständigkeit hin untersucht. Hierzu wurden zunächst die L- und E-Werte, die dem Fachmann bekannte Messgrößen darstellen, unter Verwendung entsprechend gefärbter Strähnen gemessen. Danach wurden die gefärbten Strähnen viermal bei ca. 35°C mit einem handelsüblichen Shampoo gewaschen, anschließend die L- und E-Werte erneut gemessen und die Änderung der Helligkeit (DL-Wert) und die Gesamtfarbänderung (DE-Wert) durch Differenzbildung bestimmt. Die Ergebnisse sind in Tabelle 3 aufgeführt.

**Tabelle 3**

| DL- und DE-Werte für die Farbmittel 1-5 | | |
|---|---|---|
| Farbmittel | DL-Wert | DE-Wert |
| 1 | 2,62 | 3,44 |
| 2 | 3,12 | 7,05 |
| 3 | 3,19 | 5,64 |
| 4 | 0,2 | 1,97 |
| 5 | 2,07 | 4,8 |

Die DL-Werte und DE-Werte wurden mittels eines Datacolor Mercury 2000 Farbmessgerätes nach der CIE L *a *b, D65/10° Methode ermittelt.

Die Ergebnisse der Tabelle 3 zeigen, dass sich die untersuchten Farbmittel auf Anthrachinonbasis durch hohe Waschbeständigkeit auszeichnen.

Formulierungsbeispiele (alle Prozentangaben sind in Gew.-%)

| Tönungsshampoo | | | |
|---|---|---|---|
| A | GENAGEN® CAB 818 | (Clariant) | 7,0 % |
| | Cocoamidopropyl Betain | | |
| | VELSOL™ Blue 25 | (Clariant) | 0,5 % |
| | Wasser | | 30,0 % |
| B | GENAPOL® T 500 P | (Clariant) | 0,5 % |
| | Ceteareth-50 | | |
| | Wasser | | ad 100,0 % |
| C | GENAPOL® LRO flüssig | (Clariant) | 30,0 % |
| | C_{12/14}-Alkyldiglycolethersulfat, Na-salz | | |
| | GENAGEN® LAA | (Clariant) | 3,0 % |
| | Lauroamphoacetat, Na-salze | | |
| | GENAMIN® CTAC | (Clariant) | 1,0 % |
| | Cetrimoniumchlorid | | |
| | Ethylendiamintetraacetat, Na-Salz | | 0,1 % |
| | NIPAGUARD® DMDMH | (Clariant) | 0,3 % |
| | DMDM Hydantoin | | |
| | [1,3-Bis(Hydroxymethyl)-5,5-dimethylimidazolidine-2,4-dione) | | |
| | GENAPOL® PDB | (Clariant) | 3,0 % |
| | Glycol Distearat und Laureth-4 und Cocoamidopropyl Betain | | |
| | Kaliumphosphate | | 1,5 % |
| D | Zitronensäure | | q.s. |

### Herstellweise:

- I: Lösen der Komponenten A unter Rühren
- II: Mischen der Komponenten B und Lösen unter Rühren und mildem Erwärmen
- III: Abkühlen von B auf ca. 35°C; nacheinander Zugabe der Komponenten C zu B
- IV: Einrühren der Komponenten A in B und C
- V: Einstellen auf pH 5,5 mit Komponente D

| Tönungsschaumfestiger | | | |
|---|---|---|---|
| A | Wasser | | q.s. |
| | GENAPOL® C100 | (Clariant) | 1,0 % |
| | Coceth-10 | | |
| | Polyquaternium-10 | | 0,1 % |
| | GENAMIN® CTAC | (Clariant) | 0,2 % |
| | Cetriumonium Chlorid | | |
| | GENAMIN® KDMP | (Clariant) | 0,2 % |
| | Behentrimonium Chlorid | | |
| B | Ethanol | | 3,0 % |
| | Wasser | | 5,0 % |
| | DIAFORMER® Z-651 | (Clariant) | 3,5 % |
| | Acrylates/Lauryl Acrylate/Stearyl Acrylate/Ethylamine Oxide Methacrylate | | |
| | Copolymer | | |
| | VELSOL™ Violet 126 | (Clariant) | 0,4 % |
| C | Zitronensäure | | q.s. |

### Herstellweise:

- I: Erhitzen der Komponenten A auf ca. 75 °C
- II: Lösen der Komponenten B unter Rühren
- III: Abkühlen von A auf ca. 35 °C
- IV: Zugabe von B zu A
- V: Einstellen auf pH 4,0 mit C

## Patentansprüche

1. Haarfärbemittel enthaltend eine oder mehrere Anthrachinonverbindungen der Formel I 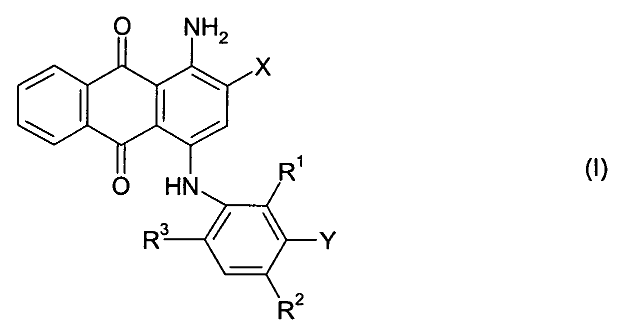 worin
R¹, R² und R³ jeweils unabhängig voneinander für Wasserstoff, für eine lineare oder verzweigte Alkylgruppe mit 1 bis 22, vorzugsweise 1 bis 8, besonders bevorzugt 1 bis 4, Kohlenstoffatomen, für eine lineare oder verzweigte Alkenylgruppe mit 2 bis 22, vorzugsweise 2 bis 8, besonders bevorzugt 2 bis 4, Kohlenstoffatomen oder für eine Gruppe N(R⁴)C(O)R⁵ stehen,
R⁴ und R⁵ unabhängig voneinander Wasserstoff oder eine Alkylgruppe mit 1 bis 22, vorzugsweise 1 bis 8, besonders bevorzugt 1 bis 4, Kohlenstoffatomen bedeuten,
X SO₃A¹ oder OCH₂CH₂(OCH₂CH₂)ₓOCH₃ bedeutet,
Y H oder SO₃A² ist,
A¹ und A² jeweils unabhängig voneinander H⁺, Li⁺, Na⁺, K⁺, Mg²⁺/2, Ca²⁺/2, Al³⁺/3 oder ⁺NR⁶R⁷R⁸R⁹, vorzugsweise H⁺, Na⁺ oder ⁺NR⁶R⁷R⁸R⁹, bedeuten,
R⁶, R⁷, R⁸, R⁹ jeweils unabhängig voneinander Wasserstoff, einen Alkylrest mit 1 bis 22, vorzugsweise 1 bis 18, Kohlenstoffatomen oder einen Hydroxyalkylrest mit 2 bis 10 Kohlenstoffatomen bedeuten, und
x für eine Zahl von 0 bis 10, vorzugsweise 1 bis 5, besonders bevorzugt 1, steht,
**dadurch gekennzeichnet, dass** die Verbindungen der Formel I genau eine Gruppe ausgewählt aus SO₃A¹ oder SO₃A² enthalten.

2. Haarfärbemittel gemäß Anspruch 1, **dadurch gekennzeichnet, dass**
R¹, R² und R³ gleich sind und für Wasserstoff oder für Methyl stehen,
X SO₃A¹ ist,
A¹ H⁺, Li⁺, Na⁺, K⁺, Mg²⁺/2, Ca²⁺/2, Al³⁺/3 oder +NR⁶R⁷R⁸R⁹, vorzugsweise H⁺, Na⁺ oder ⁺NR⁶R⁷R⁸R⁹, bedeutet,
R⁶, R⁷, R⁸, R⁹ jeweils unabhängig voneinander Wasserstoff, einen Alkylrest mit 1 bis 22, vorzugsweise 1 bis 18, Kohlenstoffatomen oder einen Hydroxyalkylrest mit 2 bis 10 Kohlenstoffatomen bedeuten, und
Y H bedeutet.

3. Haarfärbemittel gemäß Anspruch 1, **dadurch gekennzeichnet, dass**
R¹ und R³ Wasserstoff sind,
R² tert.-Butyl, n-Butyl oder -N(H)C(O)CH₃ bedeutet
X SO₃A¹ ist,
A¹ H⁺, Li⁺, Na⁺, K⁺, Mg²⁺/2, Ca²⁺/2, Al³⁺/3 oder ⁺NR⁶R⁷R⁸R⁹, vorzugsweise H⁺, Na⁺ oder ⁺NR⁶R⁷R⁸R⁹, bedeutet,
R⁶, R⁷, R⁸, R⁹ jeweils unabhängig voneinander Wasserstoff, einen Alkylrest mit 1 bis 22, vorzugsweise 1 bis 18, Kohlenstoffatomen oder einen Hydroxyalkylrest mit 2 bis 10 Kohlenstoffatomen bedeuten, und
Y H bedeutet.

4. Haarfärbemittel gemäß Anspruch 1, **dadurch gekennzeichnet, dass**
R¹, R² und R³ für Methyl stehen,
X OCH₂CH₂(OCH₂CH₂)ₓOCH₃ bedeutet,
x 1 ist,
Y SO₃A² bedeutet,
A² H⁺, Li⁺, Na⁺, K⁺, Mg²⁺/2, Ca²⁺/2, Al³⁺/3 oder ⁺NR⁶R⁷R⁸R⁹, vorzugsweise H⁺, Na⁺ oder ⁺NR⁶R⁷R⁸R⁹, bedeutet, und
R⁶, R⁷, R⁸, R⁹ jeweils unabhängig voneinander Wasserstoff, einen Alkylrest mit 1 bis 22, vorzugsweise 1 bis 18, Kohlenstoffatomen oder einen Hydroxyalkylrest mit 2 bis 10 Kohlenstoffatomen bedeuten.

5. Haarfärbemittel gemäß einem der Ansprüche 1 bis 4, **dadurch gekennzeichnet, dass** es neben der einen oder den mehreren Anthrachinonverbindungen der Formel I ein oder mehrere weitere Direktfarbstoffe enthält.

6. Haarfärbemittel gemäß einem oder mehreren der Ansprüche 1 bis 5, **dadurch gekennzeichnet, dass** es ein oder mehrere Oxidationsfarbstoffvorstufen enthält.

7. Haarfärbemittel gemäß einem oder mehreren der Ansprüche 1 bis 6, **dadurch gekennzeichnet, dass** es einen oder mehrere Carrier enthält.

8. Haarfärbemittel gemäß einem oder mehreren der Ansprüche 1 bis 7, **dadurch gekennzeichnet, dass** es eine oder mehrere perlglanzgebende Verbindungen enthält.

9. Haarfärbemittel gemäß einem oder mehreren der Ansprüche 1 bis 8, **dadurch gekennzeichnet, dass** es eine oder mehrere Verbindungen mit Glitter- oder Glanzeffekten enthält.

10. Haarfärbemittel gemäß einem oder mehreren der Ansprüche 1 bis 9, **dadurch gekennzeichnet, dass** es eine oder mehrere Verbindungen mit konditionierenden und farberhaltenden Eigenschaften enthält.

11. Haarfärbemittel gemäß einem oder mehreren der Ansprüche 1 bis 10, **dadurch gekennzeichnet, dass** es, bezogen auf die fertigen Mittel, 0,001 bis 15 Gew.-% an Anthrachinonverbindungen der Formel I enthält.

12. Haarfärbemittel gemäß einem oder mehreren der Ansprüche 1 bis 10, **dadurch gekennzeichnet, dass** es in Form einer Lösung, insbesondere einer wässrigen oder wässrig-alkoholischen Lösung, einer Emulsion, einer Dispersion, einer Creme, einem Gel oder einem Aerosolschaum oder -spray vorliegt.

13. Verwendung eines Mittels gemäß einem oder mehreren der Ansprüche 1 bis 12 zum Färben von Keratinfasern, vorzugsweise zum Färben von menschlichen Haaren.
